Europäisches Patentamt

European Patent Office

Office européen des brevets

(11) Veröffentlichungsnummer: **0 418 469 A1**

# (12) EUROPÄISCHE PATENTANMELDUNG

(21) Anmeldenummer: 90109272.6

(22) Anmeldetag: 16.05.90

(51) Int. Cl.⁵: **A61K 7/043**

(30) Priorität: 19.09.89 DE 3931237

(43) Veröffentlichungstag der Anmeldung:
27.03.91 Patentblatt 91/13

(84) Benannte Vertragsstaaten:
AT BE CH DE DK ES FR GB GR IT LI LU NL SE

(71) Anmelder: Janda, Rita
Rheingoldstrasse 4a
W-8000 München 19(DE)

(72) Erfinder: Janda, Rita
Rheingoldstrasse 4a
W-8000 München 19(DE)

(74) Vertreter: Säger, Manfred, Dipl.-Ing.
Säger & Partner Postfach 81 08 09
W-8000 München 81(DE)

(54) **Wasserverdünnbarer Nagellack.**

(57) Wasserverdünnbarer Nagellack, bestehend aus 0 bis 90 Gew.-Teilen einer lösungsmittelfreien, wäßrigen, aliphatischen Polyurethandispersion und/oder einer lösungsmittelfreien, wäßrigen Polymerdispersion von Vinylestern und/oder Acrylsäureestern und/oder Methacrylsäureestern und/oder Acrylsäureester-Copolymeren mit einem Mindestgehalt von 40 Gew.-Teilen einer der oben genannten Komponenten. Gegebenenfalls können noch folgende Gewichtsteile vorhanden sein: 0,1 bis 2 eines Verdikkungsmittels, 0,1 bis 10 eines pH-Regulators; 0,1 bis 10 eines Dispergierungsmittels und 0,1 bis 10 eines Oberflächenadditivs und 0 bis 10 eines Farbstoffpigmentes.

EP 0 418 469 A1

Die vorliegende Erfindung betrifft einen wasserverdünnbaren Nagellack.

Nagellacke werden im allgemeinen auf der Basis von physikalisch trocknenden Bindemittel hergestellt, wie beispielsweise Nitrocellulose, Kunstharze mit Weichmachern und dergl., die in organischen Lösungsmitteln gelöst werden. Sie enthalten inder Regel Farbstoffe und Pigmente, wie organische Pigmente, Fischsilber, Fischsilberersatz, Perlglanzpigmente usw. Diese Nagellacke sind aufgrund ihres hohen Lösungsmittelanteils umweltbelastend und gesundheitsschädlich.

Aufgabe der vorliegenden Erfindung ist die Schaffung eines Nagellacks, der weitgehend bzw. völlig auf umwelbelastende bzw. gesundheitsschädliche Lösungsmittel verzichten kann.

Gelöst wird diese Aufgabe durch einen wasserverdünnbaren Nagellack gemäß dem Hauptpatentanspruch. Die Unteransprüche betreffen bevorzugte Ausgestaltungen der Erfindung.

Die erfindungsgemäßen Nagellacke enthalten als Lackbasis eine Mischung aus Polyurethanen und Vinylestern und/oder Acrylsäureestern und/oder Methacrylsäureestern und/oder Acrylsäureester-Copolymeren, wobei gegebenfalls die Vinylester, Acrylsäureester, Methacrylsäureester und/oder Acrylsäureester-Copolymere bzw. Polyurethane auch allein verwendet werden.

Als Polyurethane werden Emulsionen aus lösungsmittelfreien wasserverdünnbaren aliphatischen Polyurethane verwendet. Solche Dispersionen haben im allgemeinen einen Feststoffgehalt von 30 bis 60 Gew.-%. Die Emulsionen werden in Mengen von 0 bis 90 Gew.-Teilen, bezogen auf das Gesamtgewicht der Mischung, eingesetzt, bevorzugt 5 bis 90 Gew.-Tl., besonders bevorzugt 40 bis 90 Gew.-Tl., insbesondere 40 bis 60 Gew.-Tl. und ganz besonders 45 bis 60 Gew.-Tl.

Die Dispersionen können auch Lösungsmittel enthalten, wie beispielsweise 2-N-Methylpyrrolidon, bevorzugt werden aber lösungsmittelfreie Dispersionen eingesetzt.

Als zweite Basiskomponente werden folgende Stoffe eingesetzt: Lösungsmittelfreie wässrige Emulsionen von Polyvinylestern, Polyacrylaten, Polymethacrylaten und/oder Acryl-Styrol-Copolymeren. Die Dispersionen haben im allgemeinen einen Feststoffgehalt von etwa 30 bis 60 Gew.-%. Sie werden in Mengen von 0 bis 90 Gew.-Teilen, bevorzugt 5 bis 90 Gew.-Tl., besonders bevorzugt 20 bis 60 Gew.-Tl., insbesondere 40 bis 60 Gew.-Tl. und ganz besonders 40 bis 55 Gew.-Tl. eingesetzt.

Die erfindungsgemäßen Nagellacke können weiterhin folgende Stoffe enthalten:

Verdickungsmittel in einer Menge von etwa 0,1 bis 2,0 Gew.-Teilen. Als Verdickungsmittel kommen insbesondere Polyurethan-Dispersionen in Betracht, die im Unterschied von den Polyurethan-Dispersionen der Basis-Lackkomponente keine Filmbildungseigenschaften aufweisen, sondern lediglich verdickende Eigenschaften haben.

Als pH-Regulator können beispielsweise Ammoniak, Amine, wie Triethanolamin, Natronlauge und dergl. verwendet werden. Sie werden im allgemeinen in einer Menge von 0,1 bis 10 Gew.-Tl. eingesetzt und so dosiert, daß der pH-Wert der entstehenden Mischung etwa bei 7 bis 14, vorzugsweise 7 bis 7,5 liegt.

Als Dispergierungshilfsmittel bzw. Antiabsetzmittel können Stoffe wie Bentonit, Montmorillonit und dergl. verwendet. Sie werden in Mengen von 0,1 bis 10 Gew.-Tl. eingesetzt.

Als Oberflächen-Additiv werden Mittel hinzugefügt, die die Oberflächeneigenschaften verbessern, beispielsweise die Kratzfestigkeit. Solche Mittel sind beispielsweise wässrige Polyethylen-Dispersionen, die im allgemeinen einen Feststoffgehalt von etwa 30 bis 60 Gew.-% aufweisen. Sie werden in Mengen von 0,1 bis 10 Gew.-Teilen eingesetzt, vorzugesweise in Mengen von 1 bis 6 Gew.-Teilen.

Als Farbstoffe bzw. Pigmente werden die üblicherweise für Nagellacke verwendeten Stoffe eingesetzt, wie organische Farbstoffe, Pigmentpasten, Pearlpigmente und dergleichen. Sie werden in den üblicherweise verwendeten Mengen eingesetzt, im allgemeinen von etwa 0 bis 10 Gew.-Teilen.

Der erfindungsgemäße Nagellack kann ferner die folgenden Stoffe enthalten:

Wässrige Emulsionen von Naturharzen oder Pfropfpolymeren von Naturharzen mit Acrylaten mit einem Feststoffgehalt von etwa 30 bis 60 Gew.-%. Als Naturharz ist beispielsweise Schellack geeignet. Die Dispersionen werden in Mengen von o bis 20 Gew.-Teilen eingesetzt, vorzugsweise in Mengen von etwa 0 bis 10 Gew.-%.

Lösungsmittelfreie wässrige Emulsionen von Epoxidharzen mit einem Feststoffgehalt von etwa 30 bis 60 Gew.-%. Sie werden in Mengen von etwa 0 bis 10 Gew.-Teilen, vorzugsweise in Mengen von etwa 0 bis 8 Gew.-Teilen eingesetzt.

Lösungsmittelfreie wässrige Emulsionen von Polyestern und/oder Alkydharzen mit einem Feststoffgehalt von etwa 30 bis 60 ew.-% in einer Menge von etwa 0 bis 10 Gew.-Teilen, vorzugsweise von 1 bis 8 Gew.-Teilen.

Lösungsmittel freie wässrige Melaminharz-Dispersionen mit einem Feststoffgehalt von etwa 30 bis 60 Gew.-% in einer Menge von o bis 20 Gew.-Teilen, vorzugsweise von etwa 1 bis 10 Gew.-Teilen, wie beispielsweise Hydroxymethylmethylolmelamin (HMMM).

Lösungsmittelfreie wässrige Emulsionen von Polyvinylacetat und/oder -propionat mit einem Feststoffgehalt von etwa 30 bis 60 Gew.-% in einer Menge von 0 bis 20 Gew.-Teilen, vorzugsweise von 1 bis 10 Gew.-Teilen.

Für eine wirksame Vernetzung der als Basislacke dienenden Polyurethane, Vinylester, Acrylate, Methacrylate und dergl. kann ein zusätzliches Vernetzungsmittel hinzugegeben werden, beispielsweise Aciriden. Die Vernetzer werden in Mengen von 0,1 bis 5 Gew.-Teilen, vorzugsweise von etwa 0,2 bis 2 Gew.-Teilen verwendet.

Weiterhin können die üblichen Zusatzstoffe in den üblicherweise verwendeten Mengen eingesetzt werden, wie beispielsweise Wachse, Filmbildungshilsmittel, Netz- und Dispergiermittel, Füllstoffe und dergleichen.

Als Füllstoffe eignen sich insbesondere Hohlkügelchen, beispielsweise aus Glas, blättchen- oder schuppenförmige Stoffe und dergleichen.

Die Erfindung wird nachfolgend anhand der Beispiele näher erläutert.

Beispiel 1

In einem Mischer werden nacheinander folgende Bestandteile gemischt:
45 Gew.-Teile Polyurethan-Dispersion 42 % in Wasser
27 Gew.-Teile Acrylat /Styrol-Copolymerisat 42 % in Wasser
1 Gew.-Teile Aciridin-Vernetzer
3 Gew.-Teile Schellack-Dispersion 50 % in Wasser
0,6 Gew.-Teile Entschäumer
3,5 Gew.-Teile Polyethylen-Dispersion 50 4 in Wasser
(Oberflächen-Additiv)
1 Gew.-Teile pH-Regulator
1,5 Gew.-Teile Epoxydharz-Dispersion, EP-Äquiv. 0,12 50 % in Wasser
9,9 Gew.-Teile Farbpaste rot 20 % in Wasser
7,5 Gew.-Teile Polyurethan-Verdicker
Der erhaltene Nagellack zeichnet sich durch sehr guten Glanz und sehr gute Haftung aus.

Beispiel 2

In der Rezeptur des Beispiels 1 wird das Acryl-Styrol-Copolymerisat durch eine Reinacrylat-Dispersion mit einem Feststoffgehalt von 42 Gew.-% erstzt, die übrigen Bestandteile bleiben unverändert. Der erhaltene Nagellack zeichnet sich durch sehr guten Glanz und sehr gute Haftung aus.

Beispiel 3

In einem Mischer werden folgende Bestandteile nacheinander gemischt:
34 Gew.-Teile Polyurethan-Dispersion 42 % in Wasser
51 Gew.-Teile Acrylat-Styrol-Copolymerisat 42 % in Wasser
0,5 Gew.-Teile Aciridin-Vernetzer
8,3 Gew.-Teile Polyurethan-Verdicker
1,1 Gew.-Teile pH-Regulator
4 Gew.-Teile Polyethylen-Dispersion 50 % in Wasser
0,6 Gew.-Teile Entschäumer
0,4 Gew.-Teile Pearl-Pigment
Der erhaltene Nagellack zeichnet sich durch sehr guten Glanz und sehr gute Haftung aus.

Beispiel 4

In der Rezeptur des Beispiels 3 wird das Acrylat-Styrol-Copolymerisat ersetzt durch Reinacrylat-Dispersion 42 % in Wasser. Die übrigen Betsandteile bleiben unverändert. Der erhaltene Nagellack zeichnet sich durch sehr guten Glanz und sehr gute Haftung aus.

Beispiel 5

In einem Mischer werden folgende Bestandteile nacheinander vermischt:
34 Gew.-Teile Polyurethan-Dispersion 42 % in Wasser
41 Gew.-Teile Acrylat-Styrol-Coplymerisat 42 % in Wasser
10 Gew.-Teile Alkydharz, wasserverdünnbar, 50 % in Wasser
0,6 Gew.-Teile Entschäumer
3,5 Gew.-Teile Polyethylen-Dispersion 50 4 in Wasser
1 Gew.-Teile pH-Regulator
8 Gew.-Teile Polyurethan-Verdicker
1 Gew.-Teile Aciridin-Vernetzer
0,9 Gew.-Teile Pigmentpaste in Wasser
Der erhaltene Nagellack zeichnet sich durch sehr guten Glanz und sehr gute Haftung aus.

Beispiel 6

In einem Mischer werden folgende Bestandteile nacheinander vermischt:
85 Gew.-Teile Reinacrylat-Dispersion 42 % in Wasser
0,6 Gew.-Teile Entschäumer
3,5 Gew.-Teile Polyethylen-Dispersion 50 % in Wasser
1 Gew.-Teile pH-Regulator
8 Gew.-Teile Polyurethan-Verdicker
0,3 Gew.-Teile Antiabsetzmittel
1,6 Gew.-Teile Pearlpigment

Der erhaltene Nagellack ist ein Beispiel für einen Lack ohne filmbildenden Polyurethan-Bestandteil. Er zeichnet sich durch guten Glanz aus.

Beispiel 7

In einem Mischer werden folgende Bestandteile nacheinander vermischt:
85 Gew.-Teile Polyurethan-Dispersion 42 % in Wasser
0,6 Gew.-Teile Entschäumer
3,5 Gew.-Teile Polyethylen-Dispersion, 50 % in Wasser
1 Gew.-Teile pH-Regulator
8 Gew.-Teile Polyurethan-Verdicker
0,3 Gew.-Teile Antiabsetzmittel
1,6 Gew.-Teile Pigmentpaste

Der erhaltene Nagellack ist ein Beispiel für einen Nagellack ohne Vinyl- bzw. Acrylat-Komponente. Er zeichnet sich durch sehr guten Glanz aus.

**Ansprüche**

1. Wasserverdünnbarer Nagellack, bestehend aus
0 bis 90 Gew.-Teilen einer lösungsmittelfreien, wässrigen aliphatischen Polyurethandispersion; und/oder
0 bis 90 Gew.-Teilen einer lösungsmittelfreien, wässrigen Polymerdispersion von Vinylestern und/oder Acrylsäureestern und/oder Methacrylsäureestern und/oder Acrylsäureester-Coplymeren;
mit einem Mindestgehalt von 40 Gew.-Tl einer der obigen Komponenten; und gegebenenfalls:
0,1 bis 2,0 Gew.-Teilen eines Verdickungsmittels;
0,1 bis 10 Gew.-Teilen eines pH-Regulators;
0,1 bis 10 Gew.-Teilen eines Dispergierungsmittels;
0,1 bis 10 Gew.-Teilen eines Oberflächen-Additivs und
0 bis 10 Gew.-Teilen eines Farbstoffs bzw. Pigments.

2. Nagellack nach Anspruch 1, gekennzeichnet durch einen Gehalt an 0 bis 20 Gew.-Teilen einer wässrigen Naturharz-Emulsion oder einer wässrigen Emulsion eines Pfropfpolymeren aus einem Naturharz mit Acrylsäure oder Methacrylsäure.

3. Nagellack nach Anspruch 1, gekennzeichnet durch einen Gehalt an 0 bis 10 Gew.-Teilen einer lösungsmittelfreien wässrigen Epoxidharz-Emulsion.

4. Nagellack nach Anspruch 1, gekennzeichnet durch einen Gehalt an 0 bis 10 Gew.-Teilen einer lösungsmittelfreien wässrigen Emulsion eines Polyesters oder Alkydharzes.

5. Nagellack nach Anspruch 1, gekennzeichnet durch einen Gehalt an 0 bis 20 gew.-Teilen einer lösungsmittelfreien wässrigen Melaminharz-Dispersion.

6. Nagellack nach Anspruch 1, gekennzeichnet durch einen Gehalt an 0 bis 20 Gew.-Teilen einer lösungsmittelfreien wässrigen Emulsion von Polyvinylacetat und/oder -propionat.

7. Nagellack nach Anspruch 1, gekennzeichnet durch einen Gehalt an 0,1 bis 5 Gew.-Teilen eines Vernetzungsmittels.

8. Nagellack nach Anspruch 7, dadurch gekennzeichnet, daß das Vernetzungsmittel aus Aciriden besteht.

9. Nagellack nach Anspruch 1, dadurch gekennzeichnet, daß er von 5 - 15 %, vorzugsweise 10 % H$_2$O enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2537871 (A.W. FABER-CASTEL) <br> * das ganze Dokument * <br> --- | 1, 4, 6, 9 | A61K7/043 |
| X | US-A-4126144 (PATRICIA A. DUARTE) <br> * das ganze Dokument * <br> --- | 1, 6, 9 | |
| X | EP-A-819 (ELI LILLY AND COMPANY) <br> * das ganze Dokument * <br> --- | 1, 4, 7, 9 | |
| X <br><br> A | EP-A-143480 (CLAUDIO GIULIANO DE VITA) <br> * das ganze Dokument * <br><br> --- | 1 <br><br> 2-9 | |
| A | US-A-2887116 (WILLIAM M. WOODING) <br> * das ganze Dokument * <br> --- | 1-9 | |
| A | FR-A-2605883 (JEAN MICHEL GUENASSIA) <br> * das ganze Dokument * <br> ----- | 1-9 | |

**RECHERCHIERTE SACHGEBIETE (Int. Cl.5)**

A61K

Der vorliegende Recherchenbericht wurde für alle Patentansprüche erstellt

| Recherchenort | Abschlußdatum der Recherche | Prüfer |
|---|---|---|
| BERLIN | 07 DEZEMBER 1990 | SIATOU, E. |